# EUROPÄISCHE PATENTSCHRIFT

(11) **EP 0 257 541 B1**
(45) Veröffentlichungstag und Bekanntmachung des Hinweises auf die Patenterteilung: **03.01.1996**
(21) Anmeldenummer: 87112022.6
(22) Anmeldetag: 19.08.1987
(51) Int. Cl.: C07D 219/04

(54) **Chemilumineszierende Acridin-derivate und ihre Verwendung in Lumineszenzimmunoassays**
Chemilumuniscent acridine derivatives and their use in luminescence immunoassays
Dérivés de l'acridine chimioluminescents et leur utilisation pour dosage immunologique par chimioluminescence

(30) Priorität: 22.08.1986 DE 3628573
(43) Veröffentlichungstag der Anmeldung: 02.03.1988
(60) Teilanmeldung: 94119395.5
(73) Patentinhaber: HOECHST AKTIENGESELLSCHAFT, D-65926 Frankfurt am Main (DE)
(72) Erfinder: Molz, Peter, Dr., D-6500 Mainz (DE); Skrzipczyk, Heinz-Jürgen, Dr., D-6232 Bad Soden am Taunus (DE); Lübbers, Henning, Dr., D-6231 Schwalbach am Taunus (DE); Strecker, Helmut, Dr., Verstorben (DE); Schnorr, Gerd, Dr., D-6268 Bad Vilbel (DE); Kinkel, Tonio, Dr., D-6230 Frankfurt am Main 80 (DE)

(56) Entgegenhaltungen:
- EP-A- 0 082 636
- GB-A- 1 461 877
- GB-A- 2 008 247
- CLINICAL CHEMISTRY, Band 29, Nr. 8, August 1983, Seiten 1474-1479; I. WEEKS etal.: "Acridinium esters as high-specific-activity labels in immunoassay"

## Beschreibung

Lumineszierende Verbindungen finden bereits vielseitige Anwendung. Sie werden als Indikatoren in Bioassays, Enzymimmunoassays und Lumineszenzimmunoassays eingesetzt (vgl. W.P. Collins "Alternative Immunoassays", Verlag John Wiley & Sons Ltd., Chichester, 1985), werden aber auch in Nukleinsäure-Hybridisierungsassays verwendet (vgl. J.A. Matthews et al. "Analytical Biochemistry", 151, 205-209, 1985). Außerdem werden chemilumineszierende Verbindungen bei der "flow injection analysis", in post-column-Detektoren in der Flüssigkeitschromatographie, in der Strömungsforschung und in künstlichen Lichtquellen eingesetzt.

Bei den Chemilumineszenzimmunoassays haben insbesondere zwei Strukturtypen von chemilumineszierenden Markierungssubstanzen größere Bedeutung erlangt. Es handelt sich hierbei einerseits um die Luminol- bzw. Isoluminolderivate, die bei H.R. Schroeder et al., "Methods in Enzymology", Academic Press Inc., New York, Vol. LVII, 1978, 424 ff sowie in den britischen Patenten 2 008 247 und 2 041 920, den deutschen Patentschriften 26 18 419 und 26 18 511, sowie in der europäischen Patentanmeldung 135 071 beschrieben sind. Einen Überblick über die praktische Anwendung der Isoluminolverbindungen als Lumineszenzindikatoren findet man bei W.G. Wood, J. Clin.Chem. Clin. Biochem. 22, 1984 905-918.

Andererseits haben auch Acridiniumesterverbindungen als chemilumineszierende Markierungssubstanzen Anwendung gefunden. Derartige Acridiniumester sind aus dem US-Patent 3 352 791, den britischen Patenten 1 316 363 und 1 461 877 sowie aus der europäischen Patentanmeldung 82 636 bekannt. Die Anwendung der Acridiniumester als Markierungssubstanzen in Immunoassays ist bei Weeks et al., Clin. Chem. 29/8 (1983), 1474-1479, beschrieben. Auch die Verwendung von Phenanthridiniumestern als Markierungssubstanz in Lumineszenzimmunoassays ist aus der europäischen Patentanmeldung 170 415 bereits bekannt.

Die Chemilumineszenz von Acridiniumestern kann durch Zugabe von Alkalischer H₂O₂-Lösung gestartet werden. Für den Mechanismus der Chemilumineszenz hat F. McCapra, Acc.Chem. Res. 9, 201, 1976, eine überzeugende Erklärung gegeben. Dabei ist offensichtlich die Art der Abgangsgruppe sowohl für die Lichtquantenausbeute als auch für die hydrolytische Stabilität entscheidend.

Die bisher schon bekannten Acridiniumester haben gegenüber den Luminol- und Isoluminolverbindungen den Vorteil einer höheren Lichtquantenausbeute, die auch durch an den Indikator gebundene Proteine nicht beeinträchtigt wird (vgl. Weeks et al., Clin. Chem. 29/8 (1983), 1474-1479).

Obwohl die aus der europäischen Patentanmeldung 82 636 bekannten Acridiniumphenylester bei Anregung der Chemilumineszenz durch milde Oxidationsmittel sich durch eine hohe Nachweisempfindlichkeit auszeichnen, weisen sie für die praktische Anwendung störende Nachteile auf. Vor allem ist die Phenylesterbindung in wäßrigen Systemen auch schon bei Raumtemperatur sehr labil. Hinzu kommt, daß unter den dort angegebenen Oxidationsbedingungen die Acridiniumphenylester eine Lichtemission zeigen, die erst nach etwa 10 Sekunden weitgehend, d.h. zu über 95 %, abgeklungen ist. Im Vergleich dazu haben andere nicht isotopische Assayverfahren weitaus kürzere Meßzeiten und ermöglichen dadurch einen höheren Probendurchsatz.

Aufgabe der vorliegenden Erfindung war es deshalb, neue Acridiniumderivate zur Verfügung zu stellen, die bei hoher Lichtquantenausbeute eine schnellere Reaktionskinetik aufweisen und damit kurze Meßzeiten für einen Lumineszenzimmunoassay ermöglichen
Es wurde nun gefunden, daß diese Anforderungen erfüllt werden von chemilumineszierenden Acridiniumderivaten der Formel I 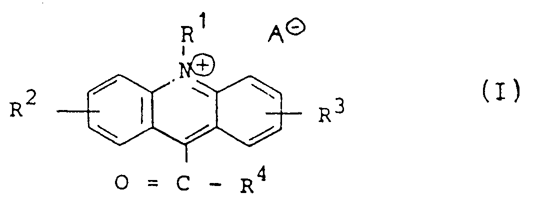
in der R¹ Wasserstoff, ein Alkyl-, Alkenyl- oder Alkinylrest mit 1 bis 10 Kohlanstoffatomen, eine Benzyl-, Phenyl- oder Naphthylgruppe ist, R² und R³ Wasserstoff, eine Alkylgruppe mit 1 bis 4 Kohlenstoffatomen, eine unsubstituierte Aminogruppe oder eine C₁-C₄ Alkyl- oder eine C₁-C₄ Dialkylaminogruppe ist, wobei die Alkylreste mit Hydroxy monosubstituiert sein können sowie zyklische Amine, wie z. B. Morpholin, eine Carboxy-, Alkoxy-, Cyano-, Nitrogruppe oder Halogen sind, R⁴ eine substituierte Sulfonamidgruppe der Formel V 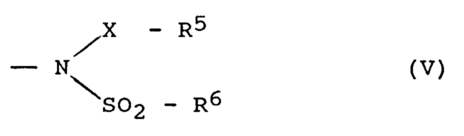
oder der Formel VI 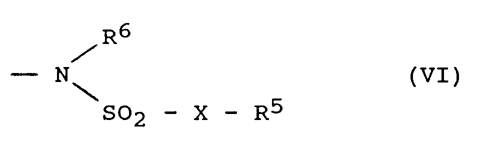
ist, oder ein Thyolalkyl- oder Thioarylrest der Formel II

- S - X - R⁵ (II)

ist, wobei X eine verzweigte oder unverzweigte C₁-C₅-Alkylengruppe oder eine Phenylen- oder Naphthylengruppe ist, die auch Heteroatome enthalten kann, und R⁵ eine reaktive Gruppe ist, die unter schonenden Bedingungen selektiv mit Amino-, Carboxy-, Thiol- oder anderen funktionellen Gruppen in Substanzen von biologischem Interesse eine Bindung eingehen kann, und A^{⊖} ein die Chemolumineszenz nicht beeinträchtigendes Anion ist, und R⁶ in der Formel V ein Alkyl- oder Alkenylrest mit 1 bis 10 Kohlenstoffatomen, eine mit C₁-C₄-Alkyl mono- oder disubstituierte Aminogruppe, eine Morpholino-, eine Benzyl- oder eine Phenyl- oder Naphthylgruppe ist, die auch mit Hydroxy, Amino, Alkoxy mit 1 bis 4 Kohlenstoffatomen oder Phenoxy- oder Naphthoxygruppen substituiert sein können und R⁶ in der Formel VI zusätzlich zu den genannten Substituenten auch noch Wasserstoff bedeuten kann.

Unter den Substanzen von biologischem Interesse sind vor allem Antigene zu verstehen. Unter diesen Begriff fallen auch Hormone, Steroide, Arzneimittel, Arzneimittelmetabolite Toxine, Alkaloide und auch Antikörper.

Das die Chemilumineszenz nicht beeinträchtigende Anion kann ein Tetrafluoroborat-, Perchlorat-, Halogenid-, Alkylsulfat-, Halosulfonat-, Alkylsulfonat- oder Arylsulfonat-Anion sein. Auch jedes andere Anion kann eingesetzt werden, sofern es die Chemilumineszenz nicht löscht oder abschwächt.

Unter Aryl werden aromatische Kohlenwasserstoffe verstanden, insbesondere Phenyl und Naphthyl. Unter Heteroatome enthaltenden aromatischen Gruppen werden aromatische Kohlenwasserstoffe verstanden, die ein Stickstoff- oder Sauerstoffatom enthalten, insbesondere Chinolin, Indol, Pyrrol und Pyridin.
Unter Halogenen werden Fluor, Chlor, Brom und Jod verstanden.

Die substituierten Aminogruppen sind bevorzugt C₁-C₄-Alkyl-oder C₁-C₄-Dialkylamine, wobei die Alkylreste ihrerseits beispielsweise mit Hydroxy monosubstituiert sein können. Ebenso kann es sich bei den substituierten Aminen um einen Morpholinrest handeln. Die für R² und R³ angegebenen Alkoxyreste sind bevorzugt solche mit 1-4 C-Atomen im Alkylteil.

Die für X angegebenen verzweigten oder unverzweigten aliphatischen Gruppen sind bevorzugt C₁-C₅-Alkylengruppen.

Im allgemeinen werden Acridiniumderivate gewählt, in denen X eine Methylen-, Äthylen-, Propylen- oder eine ortho-, meta- oder para-Phenylengruppe ist. Zur Verbesserung der Wasserlöslichkeit der Acridiniumderivate können diese Gruppen auch Heteroatome enthaltende, hydrophile Substituenten tragen.

Von besonderer Bedeutung für die Einsatzmöglichkeiten der erfindungsgemäßen Acridiniumderivate ist der Substituent R⁵. Durch die geeignete Auswahl dieser Gruppe erhält das Acridinderivat eine so hohe Reaktivität, daß es schon unter milden Bedingungen selektiv mit einer funktionellen Gruppe der nachzuweisenden biologischen Substanz eine Bindung eingehen kann. Geeignete reaktive Gruppen sind aus der folgenden Zusammenstellung zu ersehen:
a) 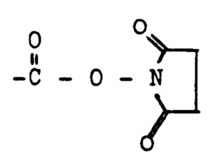
b) 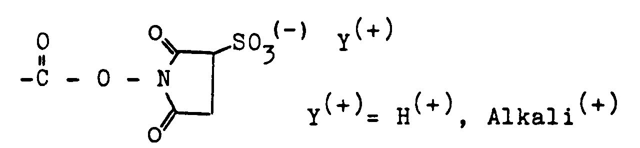
c) 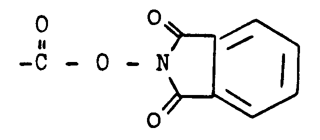
d) -SO₂ - CH = CH₂
e) 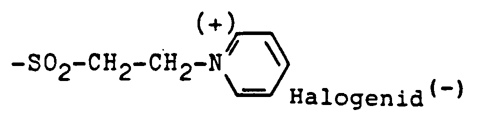
f) -N = C = S
g) 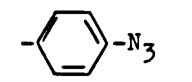
h) 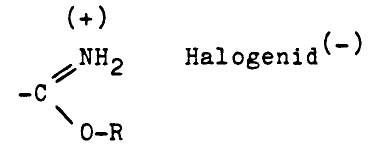
i) 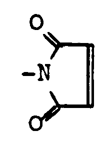
k) 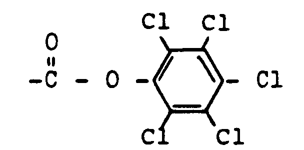
l) 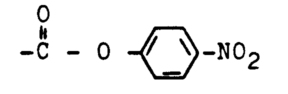
m) 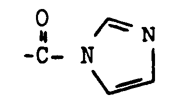
n)

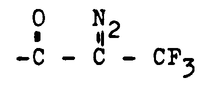

In vielen Fällen haben sich erfindungsgemäße Acridiniumderivate als geeignet erwiesen, bei denen R⁵ eine Gruppe der Formel III 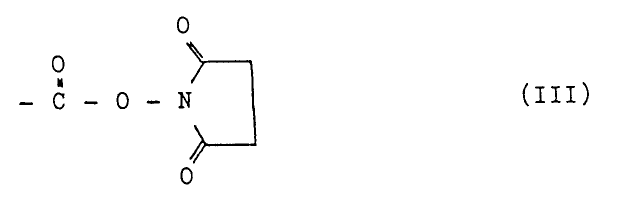
ist.

Außerdem werden auch Acridiniumverbindungen bevorzugt, in denen R¹ eine Methylgruppe ist. Besonders häufig werden deshalb erfindungsgemäße Acridiniumverbindungen eingesetzt, die der Formel IV 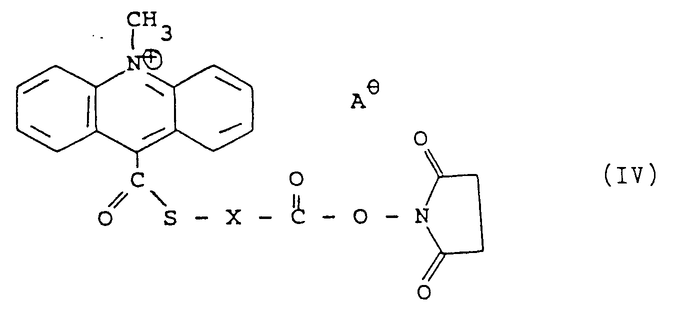
entsprechen, in denen A und X die vorstehend genannten Bedeutungen haben.

Typische Vertreter dieser erfindungsgemäßen Klasse von Acridiniumderivaten entsprechen der Formel VII 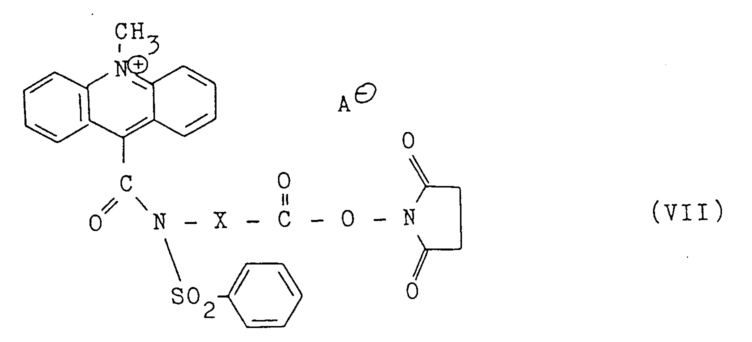
in der A und X die obengenannten Bedeutungen haben.

Durch die vorliegende Erfindung werden also zwei neue Klassen von Acridiniumverbindungen zur Verfügung gestellt, wobei die eine Klasse durch eine Thiolestergruppierung und die andere durch eine Sulfonamidstruktur sich auszeichnen. Die Acridiniumacylsulfonamid-Derivate weisen eine höhere Stabilität, die Thiolester eine schnellere Kinetik als die bisher bekannten Acridiniumphenylester-Verbindungen auf. Beide Verbindungsklassen zeichnen sich außerdem durch eine höhere Lichtausbeute aus.

Ein bedeutender Vorteil der erfindungsgemäßen Acridiniumverbindungen mit thiolesterhaltigen Abgangsgruppen gegenüber den aus der Europäischen Patentanmeldung 82 636 bekannten Acridiniumphenylestern liegt in der wesentlichen schnelleren Reaktionskinetik der Lichtemission. So liefert der nach Beispiel 1 hergestellte erfindungsgemäße Acridiniumthioester (Verbindung 6) bei 1 Sekunde Meßzeit eine um den Faktor 10 höhere Lichtausbeute unter den gleichen Oxidationsbedingungen. Diese hohe Lichtausbeute bei gleichzeitig kurzen Meßzeiten ermöglicht einen wesentlich höheren Probendurchsatz im Luminometer.

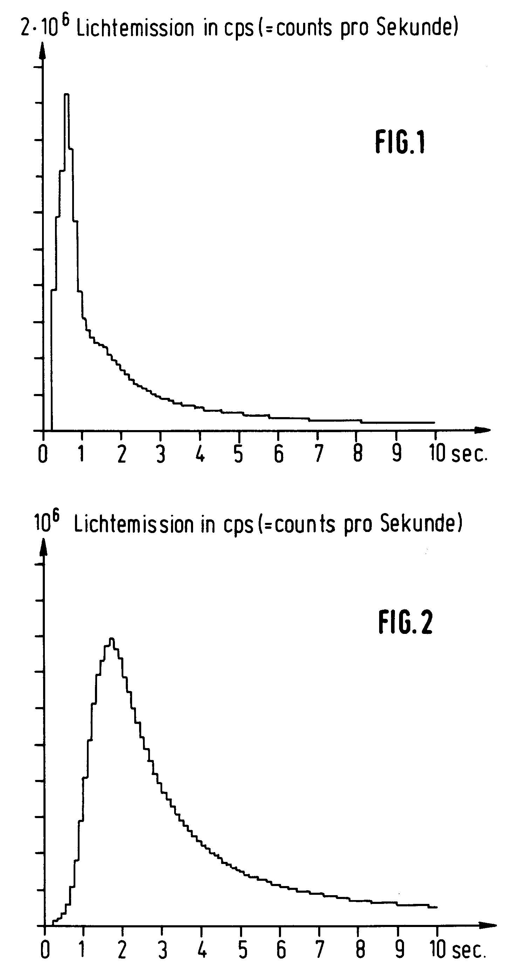

So zeigt Fig. 1 die Kinetik der Lichtemission eines Antikörperkonjugates des nach Beispiel 1 hergestellten Acridiniumthioesters (Verbindung 6)) und Fig. 2 die des Antikörperkonjugates des 4-(2-Succinimidyl-oxycarbonylethyl)-phenyl-10-methylacridinium-9-carboxylat-methosulfats (Europäische Patentanmeldung 82 636, Seite 10). 100 µl der jeweiligen Tracerlösungen werden durch Zugabe von 350 µl 0.05 m KCl/NaOH-Puffer pH 13 + 0.1 % H₂O₂ zur Chemilumineszenz angeregt und über einen Zeitraum von 10 Sekunden aufgezeichnet.

In Fig. 1 wird die maximale Lichtemission schon nach 0,66 Sekunden erreicht und ist nach 0,88 Sekunden schon wieder auf die Hälfte abgefallen. Demgegenüber wird in Fig. 2 die maximale Lichtemission erst nach 1,77 Sekunden erreicht und ist nach 2,88 Sekunden erst wieder auf die Hälfte abgefallen.

Völlig unerwartet ist, daß am Amidstickstoff sulfonylsubstituierte Acridinium-9-carbonsäureamide eine ausgezeichnete Chemilumineszenz aufweisen, denn es ist bekannt, daß Acridinium-9-carbonsäureamid in Gegensatz zu den Acridinium-9-carbonsäureestern keinerlei Chemilumineszenz zeigt (vgl. F. McCapra in W. Carruthers und J.K. Sutherland: Progress in Organic Chem., Vol. 8, 231-277, 1973, Butterworth, London).
Die erfindungsgemäßen Acridinium-9-carbonsäurethioester lassen sich auf folgendem Wege herstellen:

Acridin oder dessen Derivate mit R² und/oder R³ in den ankondensierten Phenylringen werden nach dem von Lehmstedt und Hundertmark in Ber. 63, 1229 (1930) angegebenen Verfahren in Ethanol/Eisessig und Kaliumcyanid zum 9-Cyanacridin umgesetzt. Hieraus wird nach Umkristallisation durch Umsetzung mit Schwefelsäure und Natriumnitrit entsprechend dem von Lehmstedt und Wirth in Ber. 61, 2044 (1928) beschriebenen Verfahren die Acridin-9-carbonsäure bzw. R²/R³ substituierte Acridin-9-carbonsäure gewonnen. Durch Umsetzung der Acridin-9-carbonsäure bzw. R²/R³ substituierten Acridin-9-carbonsäure mit Thionylchlorid wird die Verbindung der Formel VIII 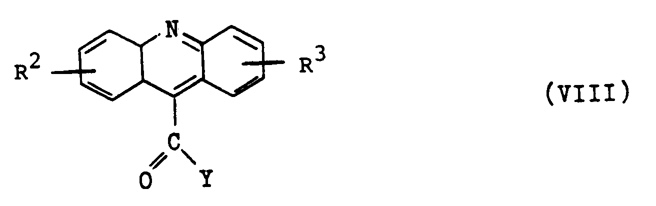
gewonnen, in der Y die Bedeutung von Chlor hat. Anstelle eines Halogens kann in die Verbindung VIII für Y auch eine Oxycarbonyl-C₁-C₅-alkyl-, Oxycarbonylaryl- oder Imidazolidgruppe eingeführt werden.

Die R²/R³-substituierten Acridinderivate können nach literaturbekannten Verfahren einfach synthetisiert werden. Solche Synthesen sind beispielsweise beschrieben in: Comprehensive Heterocyclic Chemistry; Editors A. Katritzky, C.W. Rees, Vol. 2, S. 395ff, Pergamon Press, 1984 oder Heterocyclic Compounds, Vol. 9, Acridines and Cond. 2^{nd} Edition, R.M. Acheson, John Wiley and Sons, 1973.

Das Säurechlorid (VIII) wird dann mit einer Thiolcarbonsäure der Formel IX,

HS - X - COOH (IX)

z.B. mit der 2-Mercaptobenzoesäure unter alkalischen Bedingungen zur Thiolestercarbonsäure umgesetzt, die anschließend mit einer zur Herstellung des Restes R⁵ geeigneten Verbindung, beispielsweise mit N-Hydroxysuccinimid, verestert wird. Anschließend wird die Acridinverbindung nach literaturbekannten Verfahren in 10-Stellung alkyliert. Für eine Methylierung eignet sich vor allem Trimethyloxoniumtetrafluoroborat, jedoch werden auch mit Dimethylsulfat, Methylfluorsulfonat, Toluolsulfonsäuremethylester oder Trifluoromethansulfonsäuremethylester gute Ausbeuten der chemilumineszierenden Acridiniumverbindung erhalten.

Zur Herstellung der erfindungsgemäßen Acridinium-Sulfonamidderivate wird ebenfalls vom Acridin-9-carbonsäurechlorid (VIII) ausgegangen. Diese Verbindung wird dann mit einem primären oder sekundären Sulfonamid, vorzugsweise mit einer geschützten Sulfonamidcarbonsäure der Formel X 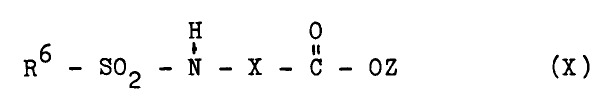
oder der Formel XI 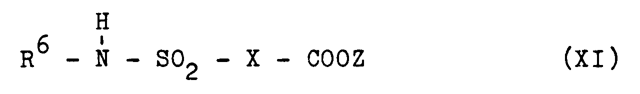
umgesetzt, in denen X und R⁶ die vorstehend genannten Bedeutungen haben und Z ein die Carboxygruppe schützender Rest ist, der anschließend abgespalten wird. Beispielsweise kann für diese Reaktion der N-Benzolsulfonylglycinbenzylester als Schutzgruppe eingesetzt werden. Die nach Abspaltung der Schutzgruppe entstehende Säure wird dann mit einer geeigneten Verbindung beispielsweise mit N-Hydroxysuccinimid, in den Rest R⁵ überführt. Hieraus wird die chemilumineszierende Acridiniumverbindung nach literaturbekannten Verfahren durch Alkylierung am Stickstoff in 10-Stellung gewonnen.

Die erhaltenen Acridiniumverbindungen lassen sich dann mit einer Substanz von biologischem Interesse, z.B. einem Antigen, einem Antikörper, einem Hormon, einem Arzneimittel, einem Arzneimittelmetaboliten, einem Toxin oder einem Alkaloid zu einer lumineszierenden Verbindung umsetzen. Dabei wird das Acridiniumderivat entweder direkt oder über ein Brückenmolekül wie Polylysin, Polyglutaminsäure oder Polyvinylamin an die biologisch interessante Substanz unter Ausbildung eines stabilen, immunologisch aktiven Konjugates gebunden. Dieses Konjugat wird auch als Tracer bezeichnet und wird in den nachfolgend beschriebenen Lumineszenzimmunoassays eingesetzt. Für den erfindungsgemäßen Lumineszenzimmunoassay zur Bestimmung einer antigenen Substanz in einer Flüssigkeitsprobe nach einem kompetitiven oder einem Sandwich-Verfahren wird mindestens eine immunologisch aktive Komponente benötigt, die auf einer festen Phase immobilisiert ist und außerdem der lumineszierende Tracer. Der Lumineszenzimmunoassay kann nun in verschiedener Weise durchgeführt werden.

Eine Möglichkeit besteht darin, daß man den mit dem Antigen spezifisch reagierenden immobilisierten Antikörper mit einer Probe der zu untersuchenden Flüssigkeit und einem Konjugat aus dem Antigen und einem chemilumineszierenden Acridiniumderivat (Antigen-Tracer) inkubiert, die Probe und den nicht gebundenen Tracer abtrennt, den gebundenen Tracer mit einem Oxidationsmittel zusammenbringt, um eine Lichtemission hervorzurufen, und dann aus der gemessenen Stärke der Lichtemission die Menge des vorhandenen Antigens bestimmt.

Eine andere Möglichkeit für die Durchführung des Lumineszenzimmunoassays besteht darin, daß man einen mit dem Antigen spezifisch reagierenden immobilisierten Antikörper mit einer Probe der zu untersuchenden Flüssigkeit, mit einem Konjugat aus einem zweiten, spezifisch reagierenden Antikörper und einem chemilumineszierenden Acridinium-Derivat inkubiert, die Probe und das nicht gebundene, markierte Konjugat abtrennt, das gebundene, markierte Konjugat mit einem Oxidationsmittel zusammenbringt, um eine Lichtemission hervorzurufen, und aus der gemessenen Stärke der Lichtemission die Menge des vorhandenen Antigens bestimmt.

Die vorstehend genannten Lumineszenzimmunoassays können auch in der Weise durchgeführt werden, das man vor der Zugabe des markierten Konjugats die zu untersuchende Flüssigkeit vom immobilisierten Antikörper abtrennt.

In anderen erfindungsgemäß durchführbaren Lumineszenzimmunoassays ist nicht der Antikörper, sondern das Antigen immobilisiert. So kann ein mit dem Antikörper spezifisch reagierendes immobilisiertes Antigen mit einer Probe der zu untersuchenden Flüssigkeit und einer Lösung eines Konjugats aus dem Antikörper und einem chemilumineszierenden Acridiniumderivat inkubiert werden, dann die Probe und das nicht gebundene markierte Konjugat abgetrennt und dann das gebundene, markierte Konjugat mit einem Oxidationsmittel zusammengebracht werden. Dann tritt eine Lichtemission auf, aus deren Stärke die Menge des vorhandenen Antigens bestimmt werden kann.

Eine weitere Variante besteht darin, daß man ein mit dem Antikörper spezifisch reagierendes, immobilisiertes Antigen mit einer Lösung eines Konjugats aus dem Antikörper und einem chemilumineszierenden Acridiniumderivat inkubiert, das nicht umgesetzte, markierte Konjugat abtrennt, eine Probe der zu untersuchenden Flüssigkeit zufügt, die Probe anschließend wieder abtrennt, das gebundene, markierte Konjugat mit einem Oxidationsmittel zusammenbringt, um eine Lichtemission hervorzurufen und aus dieser dann die Menge des vorhandenen Antigens bestimmt.

Schließlich ist der Lumineszenzimmunoassay auch in der Weise durchführbar, daß man ein mit dem Antikörper spezifisch reagierendes, immobilisiertes Antigen mit einer Lösung eines Konjugats aus dem Antikörper und einem chemilumineszierenden Acridiniumderivat inkubiert, eine Probe der zu untersuchenden Flüssigkeit zufügt, die Probe und das nicht gebundene Konjugat abtrennt, das gebundene, markierte Konjugat mit einem Oxidationsmittel zusammenbringt und dann aus der gemessenen Lichtemission die Menge des vorhandenen Antigens bestimmt.

Die Herstellung der erfindungsgemäßen Acridiniumverbindungen wird durch die Beispiele 1 bis 3 gezeigt.

### Beispiel 1

### 9-Cyanacridin (1)

Zu Acridin (10 g) in 45 ml Ethanol gibt man 3,3 ml Eisessig und tropfenweise eine Lösung von 5,25 g Kaliumcyanid in 8 ml Wasser, erhitzt die Reaktionsmischung 2 Stunden am Rückfluß, kühlt ab und zieht die flüchtigen Bestandteile im Vakuum ab. Der Rückstand wird mit 30 ml 2 N NaOH verrührt, abgesaugt und nach zweimaligem Waschen mit 2 N NaOH und Wasser einige Zeit feucht an der Luft stehengelassen. Das Rohprodukt wird in Methylenchlorid verrührt, ungelöste Substanz wird abgesaugt, und mit Methylenchlorid gewaschen, die vereinigten organischen Phasen werden eingeengt und das rohe 9-Cyanacridin wird aus n-Butylacetat umkristallisiert.
Ausbeute: 50 % Schmelzpkt.: 183-5°C
IR: 2230 cm⁻¹

### Acridin-9-carbonsäure (2)

9-Cyanacridin (5 g) wird langsam portionsweise zu 40 ml konz. H₂SO₄ gegeben und die Mischung 2 Stunden auf 90-95°C erhitzt, nach Zugabe von 8,5 g NaNO₂ wird weitere 2 Stunden bei dieser Temperatur gerührt. Die heiße Lösung wird unter schnellem Rühren auf 620 ml Eiswasser gegeben, der Niederschlag abgesaugt und in möglichst wenig 2 N NaOH gelöst. Die Lösung wird filtriert und mit 50 %iger H₂SO₄ angesäuert, die ausfallende Acridin-9-carbonsäure abgesaugt und im Vakuum betrocknet.
Ausbeute: 95 % Schmelzpkt.: 288-9°C
IR: 3440(br), 3200(br), 2600-2500(br), 1980; 1650; 1605; 1420 cm⁻¹

### Acridin-9-carbonsäurechlorid-hydrochlorid (3)

Acridin-9-carbonsäure (5 g) wird portionsweise zu 50 ml frisch destilliertem SOCl₂ gegeben und 5 h am Rückfluß erhitzt; die dann klare Lösung wird durch Destillation bis zum beginnenden Niederschlag konzentriert, die Fällung wird durch Cyclohexanzugabe und Abkühlen vervollständigt. Absaugen des Niederschlages und Trocknung im Vakuum ergibt Acridin-9-carbonsäurechloridhydrochlorid.
Ausbeute: 90 % Schmelzpkt.: 223°C

| Elementaranalyse (berechnet als C₁₄H₉ClNO x HCl) | | | | |
|---|---|---|---|---|
| ber. | C 60,5 | H 2,8 | N 5,0 | Cl 25,5 |
| gef. | C 59,4 | H 3,3 | N 5,0 | Cl 25,2 |

### (Phenyl-2ʹ-carbonsäure)acridin-9-thiocarboxylat (4)

Acridin-9-carbonsäurechlorid-hydrochlorid (30 g) wird in 720 ml Methylenchlorid suspendiert, Thiosalicylsäure (17,7 g) und 50 ml Triethylamin zugegeben, die klar werdende Lösung rührt man 10 Minuten bei Raumtemperatur nach. Nach Abziehen des Lösungsmittels wird der Rückstand mit 35 g Soda und 1400 ml Wasser versetzt, die resultierende Lösung bis zum Auftreten eines Niederschlags eingeengt, dieser wird abgesaugt. Das Filtrat wird mit NaCl gesättigt und der dabei ausfallende Niederschlag ebenfalls abgesaugt, Die wäßrige Lösung der vereinigten Niederschläge wird bei 80°C mit Eisessig angesäuert, das ausfallende Produkt abgesaugt und im Vakuum getrocknet.
Ausbeute: 80 % Schmelzpkt.: 261-5°C
NMR (DMSO, 100 MHz): δ= 7,6-8,4 ppm, komplexes Multiplett
IR: 1680 cm⁻¹ (s), 1720 (m) 1260 (s)

### 2ʹ-(Succinimidoyloxycarbonyl)phenylacridin-9-thiocarboxylat (5)

Zur Suspension von 10 g Thiolestercarbonsäure im 190 ml trockenen Tetrahydrofuran gibt man bei 0°C 3,2 g N-Hydroxysuccinimid, dann bei -20°C 6,9 g Dicyclohexylcarbodiimid (DCC) zu und läßt bei -20°C 2 Stunden, dann über Nacht bei Raumtemperatur nachrühren. Nach Zugabe von 0,28 ml Eisessig wird 1 Stunde gerührt, dann Essigester (25 ml) zugegeben und vom Niederschlag abfiltriert. Das Filtrat wird eingeengt und ergibt nach Umkristallisation aus Chlorbenzol blaßgelbes 2ʹ-(Succinimidoyloxycarbonyl)phenylacridin-9-thiocarboxylat.
Ausbeute: 80 % Schmelzpkt.: 198-200°C
IR: 1810 cm⁻¹, 1780, 1745, 1225, 1205
NMR (DMSO), 100 MHz : δ= 2,95 ppm (s, 4H), 7,7-8,4 ppm (m, 12H)

### 2ʹ-(Succinimidoyloxycarbonyl)phenyl-10-methylacridinium-9-thiocarboxylat-tetrafluoroborat (6)

3 g N-Hydroxysuccinimidester (5) werden mit 7,8 g Trimethyloxoniumtetrafluoroborat in 40 ml 1,2-Dichlorethan 8 Stunden auf 80°C erhitzt und über Nacht bei Raumtemperatur nachgerührt. Der Niederschlag wird abfiltriert und mit 1,2-Dichlorethan ausgekocht. Die vereinigten organischen Phasen werden eingeengt und aus Aceton-Diisopropylether umkristallisiert.
Ausbeute: 40 % Schmelzpkt.: 245°C
IR: 3440 cm⁻¹ (br), 1800, 1780, 1740(s), 1670, 1065(s)
NMR (DMSO, 100 MHz); δ= 3,0 ppm (s, 4H), 4,95 ppm (s, leicht verbreitert, 3H), 7,9-8,6 (m, 10H), 9,9 ppm (d, 2H)

### Beispiel 2

Die Darstellung von Succinimidoyloxycarbonylmethyl-10-methylacridinium-9-thiocarboxylat-tetrafluoroborat, ausgehend vom Säurechlorid (3) und Thioglykolsäure erfolgt analog der Synthese von (6). Die Ausbeuten der einzelnen Syntheseschritte sowie die spektroskopische Charakterisierung der Produkte (7) bis (9) sind nachfolgend angegeben:

### Carboxymethylacridin-9-thiocarboxylat (7)

Ausbeute: 60 % Schmelzpkt.: 218°C (unter Zersetzung)
IR: 3440 cm⁻¹ (br), 2400(br), 1950(br), 1710(m), 1660(s), 1070(m)
NMR (DMSO, 100 MHz): δ= 4,25 ppm (s, 2H); 7,6-8,4 (m, 8H)

### Succinimidoyloxycarbonylmethylacridin-9-thiocarboxylat (8)

Ausbeute: 80 %
IR: 3440 cm⁻¹ (br), 2930, 1820, 1785, 1740(s), 1205, 1165
NMR (DMSO, 100 MHz); δ= 2,95 ppm (s, 4H), 4,77 ppm (s, 2H), 7,6-8,3 ppm (m, 8H)

### Succinimidoyloxycarbonylmethyl-10-methylacridinium-9-thiocarboxylat-tetrafluoroborat (9)

Ausbeute: 40 % Schmelzpkt.: 250°C
IR: 3440 cm⁻¹ (br), 1810, 1780, 1735(s), 1538, 1350, 1060
NMR (DMSO, 100 MHz): δ= 2,9 ppm (s, 4H), 4,8 (s, 2H), 4,9 ppm, (s, 3H), 7,7-9,0 (m, 8H)

### Beispiel 3

### N-Benzolsulfonyl-N-(benzyloxycarbonylmethyl)acridin-9-carbonsäureamid (10)

3,3 g N-Benzolsulfonylglycinbenzylester werden in 110 ml Tetrahydrofuran mit 130 mg 4-(Dimethylamino)-Pyridin und 6 ml Triethylamin versetzt, nach 10 Minuten gibt man 3 g Acridin-9-carbonsäurechlorid-hydrochlorid zu und erhitzt die entstehende Suspension 6 Stunden zum Rückfluß. Der Niederschlag wird abgesaugt, das Lösungsmittel abgezogen, der Rückstand in Methylenchlorid aufgenommen und kurz mit 2 N NaOH verrührt. Die organische Phase wird nach Trocknen über MgSO₄ eingeengt und der resultierende Rückstand aus Toluol/Heptan umkristallisiert.
Ausbeute: 70 % Schmelzpkt.: 58°C
IR: 3440 cm⁻¹ (br), 1735, 1680, 1357, 1165
NMR (DMSO, 100 MHz): δ= 5,2 ppm (s, 2H), 5,3 ppm (s, 2H), 7,0-8,4 ppm (m, 18H)

### N-Benzolsulfonyl-N-(carboxymethyl)acridin-9-carbonsäureamid (11)

1 g N-Benzolsulfonyl-N(benzyloxycarbonylmethyl)acridin-9-carbonsäureamid in 60 ml Eisessig wird unter Zusatz von 2 ml konzentrierter HCl und Pd/C (10 %) bei Raumtemperatur und Normaldruck hydriert; nach Beendigung der Reaktion wird der Katalysator abgesaugt, aus dem Filtrat erhält man bei Einengen die Carbonsäure als gelben Feststoff.
NMR (DMSO, 100 MHz): δ= 5,0 ppm (s, 2H), 7,1-8,5 ppm (m, 13H)
Die Verbindung (11) wird mit N-Hydroxysuccinimid analog zur Darstellung von (5) zu N-Benzolsulfonyl-N-(succinimidoyloxycarbonylmethylacridin-9-carbonsäureamid (12) umgesetzt. (12) wird mit Trimethyloxoniumtetrafluoroborat, wie bei (6) beschrieben, zu N-Benzolsulfonyl-N-(succinimidoyloxycarbonylmethyl)-10-methylacridinium-9-carbonsäureamidtetrafluoroborat (13) quaternisiert.

### Beispiel 4:

### N-Phenyl-N(4-benzyloxycarbonylbenzolsulfonyl)acridin-9-carbonsäureamid (14)

11 g 4(N-Phenylsulfamido)benzoesäurebenzylester werden in 300 ml Methylenchlorid mit 360 mg 4-(Dimethylamino)pyridin und 16.6 ml Triethylamin versetzt, nach 10 Minuten gibt man 8.34 g Acridin-9-carbonsäurechlorid-hydrochlorid (3) zu und erhitzt 16 Stunden zum Rückfluß. Die abgekühlte Lösung wird kurz mit 2N NaOH verrührt, die abgetrennte organische Phase mit Wasser gewaschen, über Na₂SO₄ getrocknet und eingeengt. Der Rückstand wird aus Toluol/Heptan umkristallisiert.
Ausbeute: 70 % Schmelzpunkt 161 - 163°C
NMR(DMSO, 100 MHz): δ= 5,5 ppm (s,2H), δ= 6.8-8.6 ppm (m,22H)

### N-Phenyl-N-(4-carboxybenzolsulfonyl)acridin-9-carbonsäureamidhydrobromid (15)

8,58g N-Phenyl-N-(4-benzyloxycarbonylbenzolsulfonyl)acridin-9-carbonsäureamid (14) werden in 30 ml 33 % HBr in Eisessig 2 Stunden auf 60° C erhitzt, nach Abkühlen fügt man 60 ml Diisopropylether hinzu, saugt den Niederschlag ab und trocknet im Vakuum.
Ausbeute: 95 % Schmelzpunkt: 255°C
NMR(DMSO, 100 MHz): δ= 6.8-9 ppm (m)

### N-Phenyl-N-(4-succinimidoyloxycarbonylbenzolsulfonyl)acridin-9-carbonsäureamid (16)

5,63 g N-Phenyl-N-(4-carboxybenzolsulfonyl)acridin-9-carbonsäureamid-hydrobromid (15) in 250 ml Tetrahydrofuran werden mit 2,8 ml Triethylamin versetzt, auf -15°C abgekühlt und mit 0,96 ml Chlorameisensäureethylester versetzt. Man rührt 20 Minuten nach, gibt 1, 15 g N-Hydroxysuccinimid zu, rührt 3 Stunden bei -15°C, läßt auf Raumtemperatur auftauen und über Nacht nachrühren. Vom Niederschlag wird abgesaugt, das Filtrat eingeengt, der Rückstand mit Methylenchlorid aufgenommen, die resultierende Lösung mit Wasser, NaHCO₃-Lösung und Wasser gewaschen und über Na₂SO₄ getrocknet. Die organische Phase wird eingeengt und der Rückstand aus Toluol umkristallisiert.
Ausbeute: 50 % Schmelzpunkt 226° (Zersetzung)
NMR(DMSO, 100 MHz): δ= 2,95 ppm (s,4H), δ= 6.8-8.7 ppm (m, 17H)

### N-Phenyl-N-(4-succinimidoyloxycarbonylbenzolsulfonyl)-10-methylacridinium-9-carbonsäureamid-fluorosulfonat (17)

1,16 g N-Phenyl-N-(4-succinimidoyloxycarbonylbenzolsulfonyl)acridin-9-carbonsäureamid (16) werden in 60 ml 1,2-Dichlorethan mit 0.3 ml Methylfluorosulfonat 24 Stunden bei Raumtemperatur gerührt, der ausfallende Niederschlag wird abgesaugt und im Vakuum getrocknet.
Ausbeute: 65 %
IR: 3420 cm⁻¹ (br), 3100(br), 1805(w), 1770(m) 1745(s), 1700(m), 1385(m), 1280(m), 1255(s), 1230(s), 1205(s)
NMR(DMSO, 100 MHz): δ= 2,95 ppm (s,4H), δ= 4.75 ppm (s,br,3H), δ= 7.0-9.0 ppm (m,17H)
Massenspektrum: m/z = 594 : M⁺ (Kation)

### Beispiel 5

Die Darstellung von N-(4-Methoxyphenyl)-N-(4-succinimidoyloxycarbonylbenzolsulfonyl)-10-methylacridinium-9-carbonsäureamid-fluorosulfonat (21) ausgehend von 4-[N-(4ʹ-Methoxyphenyl)sulfamido]-benzoesäurebenzylester und Acridin-9-carbonsäurechlorid-hydrochlorid (3) erfolgt analog der Synthese von (17) (s. Beispiel 4). Die Ausbeuten der einzelnen Syntheseschritte sowie die spektroskopische Charakterisierung sind nachfolgend angegeben.

### N-(4-Methoxyphenyl)-N-(4-benzyloxycarbonyl-benzolsulfonyl)acridin-9-carbonsäureamid (18)

Ausbeute: 70 % Schmelzpunkt: 182-183°C
NMR(DMSO, 100 MHz): δ= 3,5 ppm (s, 3H), δ= 5,5 ppm (s,2H), δ= 6.35-6.63 ppm (d,br,2H), δ= 7.05-7.2 ppm (d,br,2H), δ= 7.35-8.5 ppm (m,17H)

### N-(4-Methoxyphenyl)-N-(4-carboxybenzolsulfonyl)acridin-9-carbonsäureamid-hydrobromid (19)

Ausbeute: 95 % Schmelzpunkt: 273°C (Zersetzung)
NMR(DMSO, 100 MHz): δ= 3.5 ppm (s,3H), δ= 6.4-6.6 ppm (d,br,2H), δ= 7.05-7.2 ppm (d,br,2H), δ= 7.7-8.5 ppm (m,12H)

### N-(4-Methoxyphenyl)-N-(4-succinimidoyloxycarbonylbenzolsulfonyl)acridin-9-carbonsäureamid (20)

Ausbeute: 50 % Schmelzpunkt: 232-234°C
NMR(DMSO,100 MHz): δ= 2,95 ppm (s,4H), δ= 3.5 ppm (s,3H), δ= 6.4-6.6 ppm (d,br,2H), δ=7.05-7.25 ppm (d,br,2H), δ= 7.8-8.6 ppm (m,12H) IR: 3050 cm⁻¹ 1805(w), 1780(m), 1740(s), 1700(m), 1505(m), 1370(m), 1250(m), 1200(s), 1185

### N-(4-Methoxyphenyl)-N-(4-succinimidoyloxycarbonylbenzolsulfonyl)-10-methylacridinium-9-carbonsäureamid-fluorosulfonat (21)

Die Substanz fällt bei der Reaktion nicht aus, sie wird durch Einengen der Lösung und Verrühren des Rückstandes mit Diisopropylether gewonnen.
Ausbeute: 80 %
NMR(DMSO, 100 MHz): δ= 2,95 ppm (s,4H), δ= 3,5 ppm (s,3H), δ= 4.8 ppm (s,br,3H), δ= 6.45-6.7 ppm (d,br,2H), δ= 7.2-7.4 ppm (d,br,2H), δ= 7.7-9 ppm (m,12H)
Massenspektrum: m/z= 624 M⁺ (Kation)
IR: 3440 cm⁻¹ (br), 3100, 2950, 1805(w), 1775(m), 1740(s), 1695(m), 1610(m), 1505(m), 1375(m), 1280(m), 1250(s), 1205(s).

### Beispiel 6

Die Darstellung von N-(4-Methoxyphenyl)-N-(3-succinimidoyloxycarbonyl-benzolsulfonyl)-10-methylacridinium-9-carbonsäureamid-fluorosulfonat(25) ausgehend vom 3-[N-(4ʹ-Methoxyphenyl)sulfamido]-benzoesäurebenzylester und Acridin-9-carbonsäurechlorid-hydrochlorid (3) erfolgt analog zur Synthes von (17) (s. Beispiel 4). Die Ausbeute der einzelnen Syntheseschritte sowie die spektroskopische Charakterisierung sind nachfolgend angegeben.

### N-(4-Methoxyphenyl)-N(3-benzyloxycarbonylbenzolsulfonyl)acridin-9-carbonsäureamid (22)

Ausbeute: 70 % Schmelzpunkt 168-170°C
NMR(DMSO, 100 MHz): δ= 3.5 ppm (s,3H), δ=5.45 ppm (s,2H), δ= 6.5 ppm (s,br,2H), δ= 7.1 ppm (br,2H), δ= 7.3-8.8 ppm (m,17H)

### N-(4-Methoxyphenyl)-N-(3-carboxybenzolsulfonyl)acridin-9-carbonsäureamid-hydrobromid (23)

Ausbeute: 90 % Schmelzpunkt: 264°C
NMR(DMSO, 100 MHz): δ= 3.5 ppm (s,3H), δ= 6.4-6.6 ppm (d,br,2H), δ=7.0-7.2 ppm (d,br,2H), δ= 7.6-8.8 ppm (m,12H)

### N-(4-Methoxyphenyl)-N-(3-succinimidoyloxycarbonylbenzolsulfonyl)acridin-9-carbonsäureamid (24)

Ausbeute: 50 % Schmelzpunkt: 223-225°C
NMR (DMSO, 100 MHz): δ= 2.95 ppm (s,4H), δ= 3.5 ppm (s,3H), δ=6.4-6.6 ppm (d,br,2H), δ= 7.0-7.2 ppm (d,br,2H), δ= 7.4-8.9 ppm (m,12H)
IR: 3500 cm⁻¹ (br), 3060, 2950, 2840, 1805(w), 1785(m), 1740(s), 1700(m), 1510(m), 1380(m), 1250(m), 1205(m), 1165(m)

### N-(4-Methoxyphenyl)-N-(3-succinimidoyloxycarbonylbenzolsulfonyl)-10-methylacridinium-9-carbonsäureamidfluorosulfonat (25)

Ausbeute: 90 %
NMR(DMSO, 100 MHz): δ= 2.95 ppm (s, 4H), δ= 3.55 ppm (s,3H), δ= 4.8 ppm (s,br,3H), δ= 6.45-6.7 (d,br,2H), δ= 7.05-7.3 ppm (d,br,2H), δ= 7.5-8.9 ppm (m,12H)
IR: 3500 cm⁻¹ (br), 3080, 2950, 1805(w), 1780(m), 1740(s),35 1700(m), 1610(m), 1510(m), 1380(m), 1250(s), 1205(s), 1170(s)
Masse: m/z= 624 M⁺ (Kation)

### Beispiel 7

### Tracer-Herstellung für den α-Fetoprotein-Chemilumineszenzimmunoassay

100 µl Antikörper (1 mg/ml), 11,5 µl des nach Beispiel 1 hergestellten Acridiniumthioesters (Verbindung (6)) (1 mg/ml in DMSO und 600 µl Konjugationspuffer (0.01 M Phosphat, pH 8.0)) werden 15 Minuten inkubiert. Danach werden 200 µl Lysin (10 mg/ml) zugefügt und weitere 15 Minuten inkubiert. Dieser Ansatz wird auf eine PD 10-Säule (Sephadex G 25 Medium, 0.1 M Phosphat, pH 6.3 als Fließmittel) transferiert. 10 Tropfen/Fraktion werden gesammelt. Die einzelnen Fraktionen werden nach geeigneter Verdünnung auf ihre Chemilumineszenzaktivität getestet (350 µl Oxidationsmittel: 0.1 % H₂O₂ in 0.1 N NaOH). Die Tracerfraktionen (1. Aktivitätspeak) werden gepoolt und bei 4°C gelagert.

### Beispiel 8

### Durchführung des α-Fetoprotein Chemilumineszenzimmunoassays

50 µl Standard/Probe und 150 µl Puffer (Phosphat; 0.1 M pH 6.3, 1 % Tween 20, 0.1 % Rinderserumalbumin, 0.1 M NaCl, 0.01 % NaN₃) werden 15 Minuten in mit monoklonalem Anti-AFP-Antikörper beschichteten Röhrchen geschüttelt. Danach wird 2 x mit 1 ml Puffer gewaschen.
200 µl Tracer werden in geeigneter Verdünnung zugeführt und 15 Minuten geschüttelt. Erneut wird 2 x mit 1 ml Puffer gewaschen. Die Chemilumeszenz-Messung wird mit 350 µl Oxidationsmittel (0.1 % H₂O₂ in 0.1 N NaOH, 2 sec. Meßzeit) gestartet.

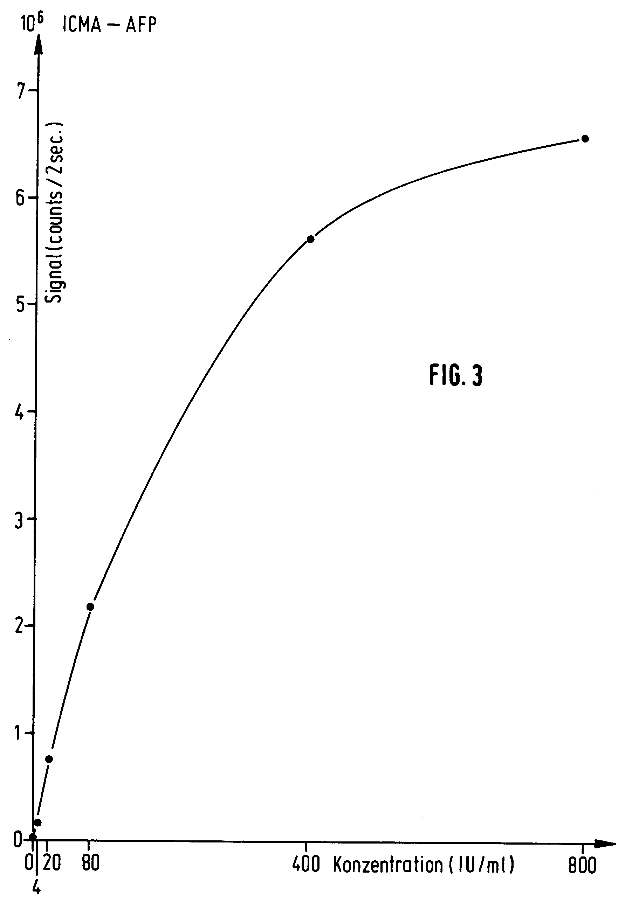

Figur 3 zeigt den typischen Verlauf einer Standardkurve eines Immuno-chemiluminometrischen Assays (ICMA) für das Alfa-Fetoprotein (AFP).

## Patentansprüche

1. Chemilumineszierendes Acridiniumderivat der Formel I 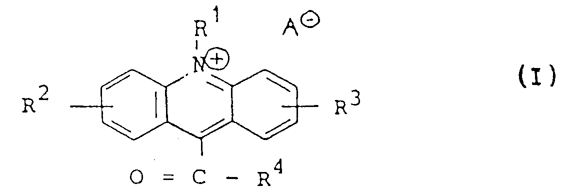 in der R¹ Wasserstoff, ein Alkyl-, Alkenyl- oder Alkinylrest mit 1 bis 10 Kohlanstoffatomen, eine Benzyl-, Phenyl- oder Naphthylgruppe ist,
R² und R³ Wasserstoff, eine Alkylgruppe mit 1 bis 4 Kohlenstoffatomen, eine unsubstituierte Aminogruppe oder eine C₁-C₄ Alkyl- oder eine C₁-C₄ Dialkylaminogruppe ist, wobei die Alkylreste mit Hydroxy monosubstituiert sein können sowie zyklische Amine, wie z. B. Morpholin, eine Carboxy-, C₁-C₄-Alkoxy-, Cyano-, Nitrogruppe oder Halogen sind,
R⁴ eine substituierte Sulfonamidgruppe der Formel V 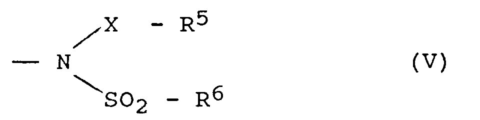 oder der Formel VI 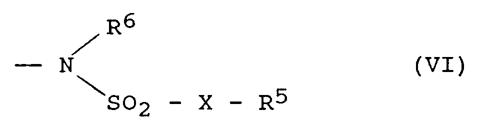 ist, oder ein Thyolalkyl- oder Thioarylrest der Formel II
- S - X - R⁵ (II)
ist, wobei X eine verzweigte oder unverzweigte C₁-C₅-Alkylengruppe oder eine Phenylen- oder Naphthylengruppe ist oder Chinol, Indol, Pyrrol oder Pyridin ist.
R⁵ eine reaktive Gruppe ist, die unter schonenden Bedingungen selektiv mit Amino-, Carboxy-, Thiol- oder anderen funktionellen Gruppen in Substanzen von biologischem Interesse eine Bindung eingehen kann, und A⁰ ein die Chemolumineszenz nicht beeinträchtigendes Anion ist, und
R⁶ in der Formel V ein Alkyl- oder Alkenylrest mit 1 bis 10 Kohlenstoffatomen, eine mit C₁-C₄-Alkyl mono- oder disubstituierte Aminogruppe, eine Morpholino-, eine Benzyl- oder eine Phenyl- oder Naphtylgruppe ist, die ggf. mit Hydroxy, Amino, Alkoxy mit 1 bis 4 Kohlenstoffatomen oder Phenoxy- oder Naphtoxygruppen substituiert ist, und
R⁶ in der Formel VI zusätzlich zu den genannten Substituenten auch noch Wasserstoff bedeuten kann.

2. Acridiniumderivat nach Anspruch 1, dadurch gekennzeichnet, daß X eine Methylen-, Ethylen-, Propylen-Gruppe oder eine ortho-, meta- oder para-Phenylgruppe ist.

3. Acridiniumderivat nach Anspruch 1, dadurch gekennzeichnet, daß R⁵ eine Gruppe der Formel III 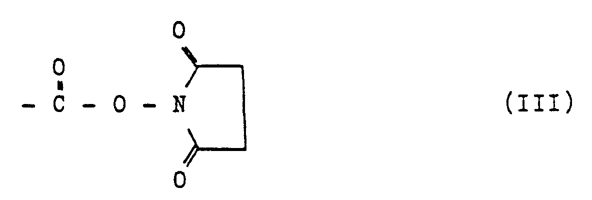 ist.

4. Acridiniumderivat nach Anspruch 3, gekennzeichnet durch die Formel IV 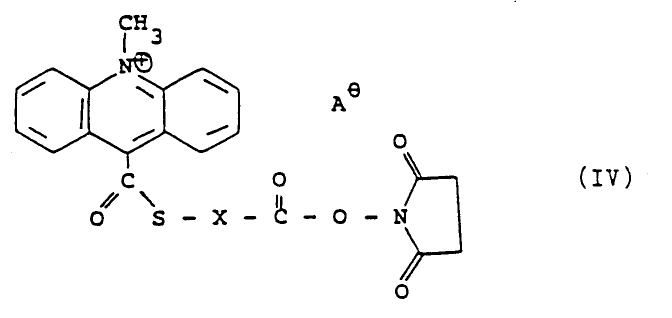 wobei A und X die in Anspruch 1 genannten Bedeutungen haben.

5. Acridiniumderivat nach Anspruch 4, gekennzeichnet durch die Formel VII 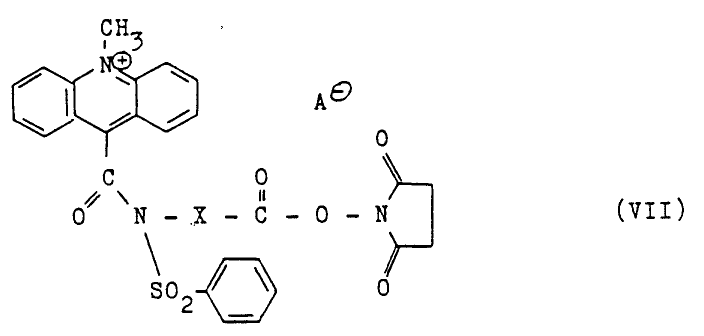 wobei A und X die in Anspruch 1 genannten Bedeutungen haben.

6. Verfahren zur Herstellung von Acridiniumderivaten gemäß Anspruch 1, dadurch gekennzeichnet, daß man eine Acridinverbindung der Formel VIII 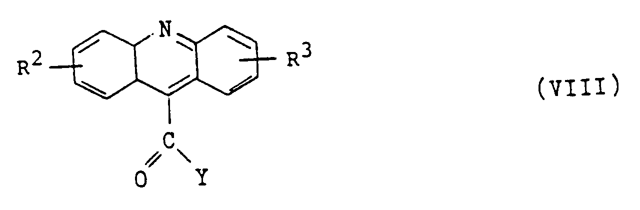 in der Y ein Halogen, eine Oxycarbonyl-C₁-C₅-alkyl-, Oxycarbonylaryl- oder Imidazolidgruppe bedeutet, zunächst mit einem primären oder sekundären Sulfonamid oder mit einer Thiolcarbonsäure der Formel IX
HS-X-COOH (IX)
umsetzt, wobei X die in Anspruch 1 genannten Bedeutungen hat, und dann die erhaltene Säure zum den Rest R⁵ enthaltenden, gewünschten Acridinderivat umsetzt, das anschließend noch am Stickstoff des Acridingerüstes quarternisiert wird.

7. Verfahren zur Herstellung von Verbindungen mit substituierten Sulfonamidgruppen der Formel V gemäß Anspruch 1, dadurch gekennzeichnet, daß man eine Verbindung der Formel VIII mit einer geschützten Sulfonamid-carbonsäure der Formel X 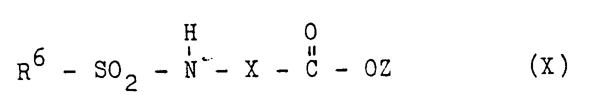 oder der Formel XI 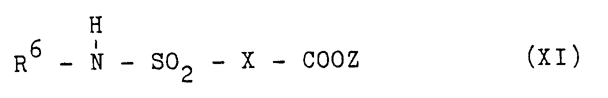 umsetzt, wobei X und R⁶ die in Anspruch 1 genannten Bedeutungen haben und Z ein die Carboxygruppe schützender Rest ist, der anschließend abgespalten wird, und die dabei entstehende Säure in das den Rest R⁵ enthaltende gewünschte Acridiniumderivat umgesetzt wird, das dann noch am Stickstoff des Acridingerüstes quaternisiert wird.

8. Lumineszente Verbindung, dadurch gekennzeichnet, daß ein Acridiniumderivat gemäß Anspruch 1 direkt oder über ein Brückenmolekül an ein Protein, ein Polypeptid oder an eine andere Substanz von biologischem Interesse unter Ausbildung eines stabilen, immunologisch aktiven Konjugates gebunden ist.

9. Lumineszenzimmunoassay zur Bestimmung einer antigenen Substanz in einer Flüssigkeitsprobe, bei dem in einem kompetitiven- oder einem Sandwich-Verfahren mindestens eine immunologisch aktive Komponente auf einer festen Phase immobilisiert ist und mindestens eine weitere Komponente, der Tracer, eine lumineszente Verbindung entsprechend Anspruch 8 darstellt.

10. Lumineszenzimmunoassay nach Anspruch 9, dadurch gekennzeichnet, daß man
a) einen mit einem Antigen spezifisch reagierenden, immobilisierten Antikörper mit einer Probe der zu untersuchenden Flüssigkeit und einem Konjugat aus dem Antigen und einem chemilumineszierenden Acridiniumderivat nach Anspruch 1 inkubiert;
b) die Probe und das nichtgebundene, markierte Konjugat abtrennt;
c) das gebundene, markierte Konjugat mit einem Oxidationsmittel zusammenbringt, um eine Lichtemission hervorzurufen und
d) aus der gemessenen Stärke der Lichtemission die Menge des vorhandenen Antigens bestimmt.

11. Lumineszenzimmunoassay nach Anspruch 10, dadurch gekennzeichnet, daß vor der Zugabe des markierten Konjugates die zu untersuchende Flüssigkeit von immobilisiertem Antikörper abgetrennt wird.

12. Lumineszenzimmunoassay nach Anspruch 9, dadurch gekennzeichnet, daß man
a) einen mit dem Antigen spezifisch reagierenden, immobilisierten Antikörper mit einer Probe der zu untersuchenden Flüssigkeit und einem Konjugat aus einem zweiten, spezifisch reagierenden Antikörper und einem chemilumineszierenden Acridiniumderivat nach Anspruch 1 inkubiert;
b) die Probe und das nichtgebundene, markierte Konjugat abtrennt;
c) das gebundene, markierte Konjugat mit einem Oxidationsmittel zusammenbringt, um eine Lichtemission hervorzurufen und
d) aus der gemessenen Stärke der Lichtemission die Menge des vorhandenen Antigens bestimmt.

13. Lumineszenzimmunoassay nach Anspruch 12, dadurch gekennzeichnet, daß vor der Zugabe des markierten Konjugates die zu untersuchende Flüssigkeit vom immobilisierten Antikörper abgetrennt wird.

14. Lumineszenzimmunoassay nach Anspruch 9, dadurch gekennzeichnet, daß man
a) ein mit dem Antikörper spezifisch reagierendes, immobilisiertes Antigen mit einer Probe der zu untersuchenden Flüssigkeit und einer Lösung eines Konjugats aus dem Antikörper und einem chemilumineszierenden Acridiniumderivat nach Anspruch 1 inkubiert;
b) die Probe und das nichtgebundene, markierte Konjugat abtrennt;
c) das gebundene, markierte Konjugat mit einem Oxidationsmittel zusammenbringt, um eine Lichtemission hervorzurufen und
d) aus der gemessenen Stärke der Lichtemission die Menge des vorhandenen Antigens bestimmt.

15. Lumineszenzimmunoassay nach Anspruch 9, dadurch gekennzeichnet, daß man
a) ein mit dem Antikörper spezifisch reagierendes, immobilisiertes Antigen mit einer Lösung eines Konjugats aus dem Antikörper und einem chemilumineszierenden Acridiniumderivat nach Anspruch 1 inkubiert;
b) das nicht umgesetzte, markierte Konjugat abtrennt;
c) eine Probe der zu untersuchenden Flüssigkeit zufügt;
d) die Probe anschließend wieder abtrennt;
e) das gebundene, markierte Konjugat mit einem Oxidationsmittel zusammenbringt, um eine Lichtemission hervorzurufen und
f) aus der gemessenen Stärke der Lichtemission die Menge des vorhandenen Antigens bestimmt.

16. Lumineszenzimmunoassay nach Anspruch 9, dadurch gekennzeichnet, daß man
a) ein mit dem Antikörper spezifisch reagierendes, immobilisiertes Antigen mit einer Lösung eines Konjugats aus dem Antikörper und einem chemilumineszierenden Acridiniumderivat nach Anspruch 1 inkubiert;
b) eine Probe der zu untersuchenden Flüssigkeit zufügt;
c) die Probe und das nichtgebundene Konjugat abtrennt;
d) das gebundene, markierte Konjugat mit einem Oxidationsmittel zusammenbringt, um eine Lichtemission hervorzurufen und
e) aus der gemessenen Stärke der Lichtemission die Menge des vorhandenen Antigens bestimmt.

## Claims

1. A chemiluminescent acridinium derivative of the formula I 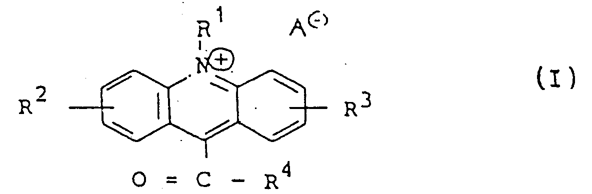 in which R¹ is hydrogen, an alkyl, alkenyl or alkynyl radical with 1 to 10 carbon atoms or a benzyl, phenyl or naphthyl group
R² and R³ are hydrogen, an alkyl group with 1 to 4 carbon atoms, an unsubstituted amino group or a C₁-C₄ alkyl- or a C₁-C₄ dialkylamino group, where the alkyl radicals can be monosubstituted by hydroxyl, and cyclic amines such as morpholine, a carboxyl, C₁-C₄-alkoxy, cyano or nitro group or halogen,
R⁴ is a substituted sulfonamide group of the formula V 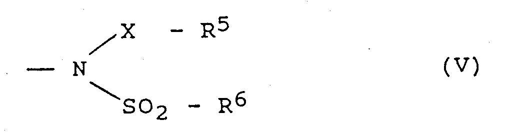 or of the formula VI 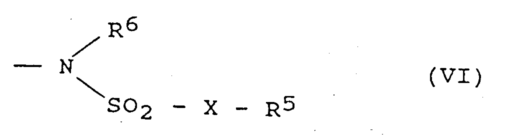 or is a thioalkyl or thioaryl radical of the formula II
- S - X - R⁵ (II)
in which X is a branched or unbranched C₁-C₅-alkylene group or a phenylene or naphthylene group or is quinole, indole, pyrrole or pyridine.

2. An acridinium derivative as claimed in claim 1, wherein X is a methylene, ethylene or propylene group or an ortho-, meta- or para-phenylene group.

3. An acridinium derivative as claimed in claim 1, wherein R⁵ is a group of the formula III

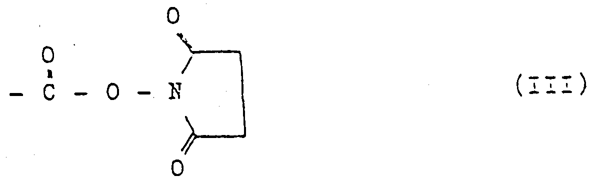

4. An acridinium derivative as claimed in claim 3, which has the formula IV 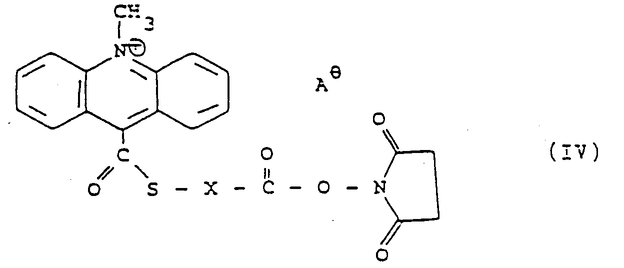 in which A and X have the meanings given in claim 1.

5. An acridinium derivative as claimed in claim 4, which has the formula VII 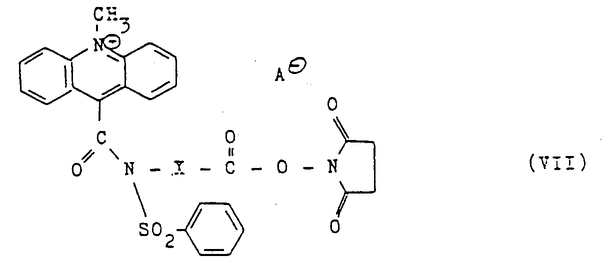 in which A and X have the meanings given in claim 1.

6. A process for the preparation of an acridinium derivative as claimed in claim 1, which comprises first reacting an acridine compound of the formula VIII 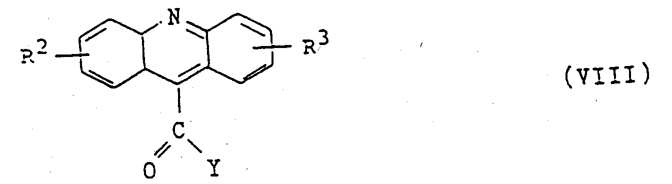 in which Y denotes a halogen or an oxycarbonyl-C₁-C₅-alkyl, oxycarbonylaryl or imidazolide group, with a primary or secondary sulfonamide or with a thiol-carboxylic acid of the formula IX
HS - X - COOH (IX)
in which X has the meanings given in claim 1, and then converting the resulting acid into the desired acridine derivative containing the radical R⁵, which is subsequently also quaternized on the nitrogen of the acridine skeleton.

7. A process for the preparation of a compound with substituted sulfonamide groups of the formula V as claimed in claim 1, which comprises reacting a compound of the formula VIII with a protected sulfonamide-carboxylic acid of the formula X 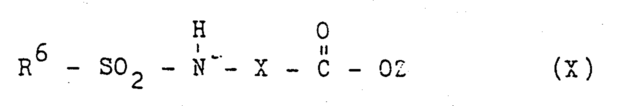 or of the formula XI 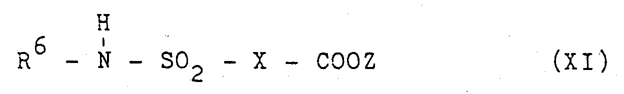 in which X and R⁶ have the meanings given in claim 1 and Z is a radical which protects the carboxyl group and is subsequently split off, and converting the acid thereby formed into the desired acridinium derivative containing the radical R⁵, which is then also quaternized on the nitrogen of the acridine skeleton.

8. A luminescent compound, wherein an acridinium derivative as claimed in claim 1 is bonded directly or via a bridge molecule to a protein, a polypeptide or another substance of biological interest to form a stable immunologically active conjugate.

9. Luminescence immunoassay for the determination of an antigenic substance in a liquid sample, in which, in a competitive process or a sandwich process, at least one immunologically active component is immobilized on a solid phase and at least one other component, the tracer, is a luminescent compound as claimed in claim 8.

10. Luminescence immunoassay as claimed in claim 9, which comprises
a) incubating an immobilized antibody which reacts specifically with an antigen with a sample of the liquid under investigation and a conjugate of the antigen and a chemiluminescent acridinium derivative as claimed in claim 1;
b) separating off the sample and the non-bonded marked conjugate;
c) bringing the bonded marked conjugate together with an oxidizing agent in order to cause photoemission and
d) determining the amount of antigen present from the photoemission intensity measured.

11. Luminescence immunoassay as claimed in claim 10, wherein the liquid under investigation is separated off from the immobilized antibody before addition of the marked conjugate.

12. Luminescence immunoassay as claimed in claim 9, which comprises
a) incubating an immobilized antibody which reacts specifically with the antigen with a sample of the liquid under investigation and a conjugate of a second antibody which reacts specifically and a chemiluminescent acridinium derivative as claimed in claim 1;
b) separating off the sample and the non-bonded marked conjugate;
c) bringing the bonded marked conjugate together with an oxidizing agent in order to cause photoemission and
d) determining the amount of antigen present from the photoemission intensity measured.

13. Luminescence immunoassay as claimed in claim 12, wherein the liquid under investigation is separated off from the immobilized antibody before addition of the marked conjugate.

14. Luminescence immunoassay as claimed in claim 9, which comprises
a) incubating an immobilized antigen which reacts specifically with the antibody with a sample of the liquid under investigation and a solution of a conjugate of the antibody and a chemiluminescent acridinium derivative as claimed in claim 1;
b) separating off the sample and the non-bonded marked conjugate;
c) bringing the bonded marked conjugate together with an oxidizing agent in order to cause photoemission and
d) determining the amount of antigen present from the photoemission intensity measured.

15. Luminescence immunoassay as claimed in claim 9, which comprises
a) incubating an immobilized antigen which reacts specifically with the antibody with a solution of a conjugate of the antibody and a chemiluminescent acridinium derivative as claimed in claim 1;
b) separating off the unreacted marked conjugate;
c) adding a sample of the liquid under investigation;
d) subsequently separating off the sample again;
e) bringing the bonded marked conjugate together with an oxidizing agent in order to cause photoemission and
f) determining the amount of antigen present from the photoemission intensity measured.

16. Luminescence immunoassay as claimed in claim 9, which comprises
a) incubating an immobilized antigen which reacts specifically with the antibody with a solution of a conjugate of the antibody and a chemiluminescent acridinium derivative as claimed in claim 1;
b) adding a sample of the liquid under investigation;
c) separating off the sample and the non-bonded conjugate;
d) bringing the bonded marked conjugate together with an oxidizing agent in order to cause photoemission and
e) determining the amount of antigen present from the photoemission intensity measured.

## Revendications

1. Dérivé d'acridinium chimiluminescent de formule I 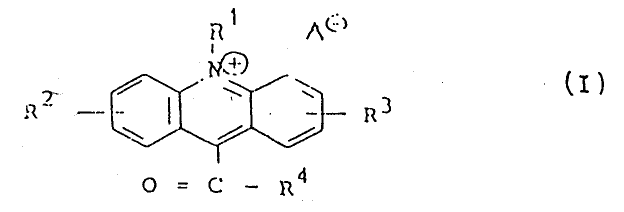 dans laquelle
R¹ est un atome d'hydrogène, un radical alkyle, alcényle ou alcynyle ayant de 1 à 10 atomes de carbone, le groupe benzyle, phényle ou naphtyle,
R² et R³ représentent un atome d'hydrogène ou d'halogène, ou un groupe alkyle ayant de 1 à 4 atomes de carbone, le groupe amino non substitué ou un groupe alkyl(C₁-C₄)- ou dialkyl(C₁-C₄)-amino, les radicaux alkyle pouvant être monosubstitués par le groupe hydroxy, ainsi que des amines cycliques, comme par exemple la morpholine, un groupe carboxy, alcoxy en C₁-C₄, cyano ou nitro,
R⁴ est un groupe sulfonamido substitué de formule V 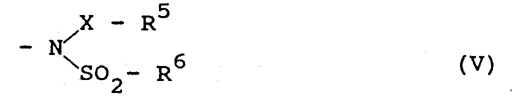 ou de formule VI 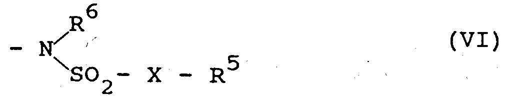 ou un radical thioalkyle ou thioaryle de formule II
- S - X - R⁵ (II)
X étant un groupe alkylène en C₁-C₅ ramifié ou non ramifié ou le groupe phénylène ou naphtylène, ou le cycle quinoléine, indole, pyrrole ou pyridine.

2. Dérivé d'acridinium selon la revendication 1, caractérisé en ce que X est le groupe méthylène, éthylène, propylène ou un groupe o-, m- ou p-phénylène.

3. Dérivé d'acridinium selon la revendication 1, caractérisé en ce que R⁵ est un groupe de formule III

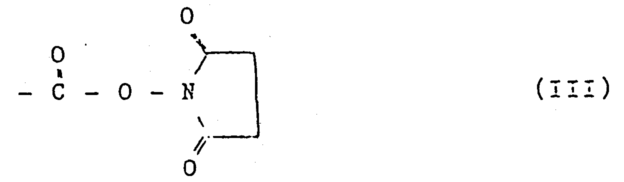

4. Dérivé d'acridinium selon la revendication 3, caractérisé par la formule IV 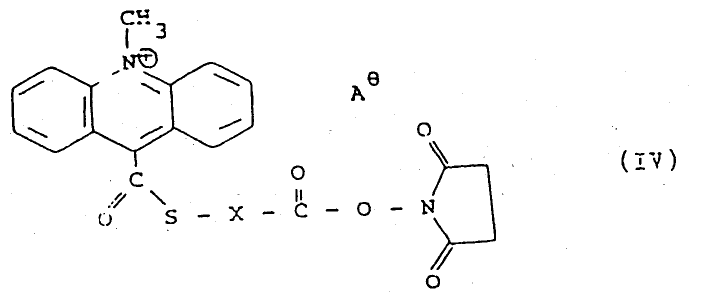 dans laquelle A et X ont les significations données dans la revendication 1.

5. Dérivé d'acridinium selon la revendication 4, caractérisé par la formule VII 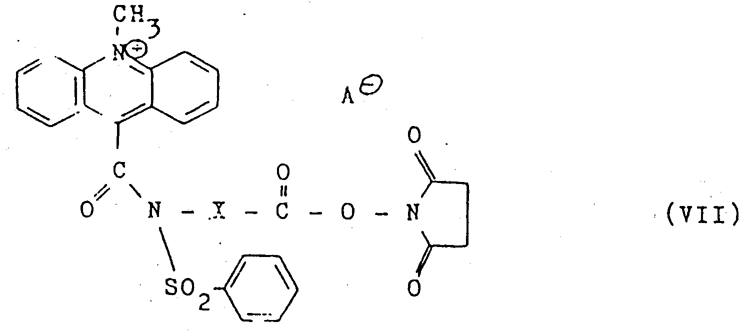 dans laquelle A et X ont les significations données dans la revendication 1.

6. Procédé pour la préparation des dérivés d'acridinium selon la revendication 1, caractérisé en ce que l'on fait d'abord réagir un composé de type acridine de formule VIII 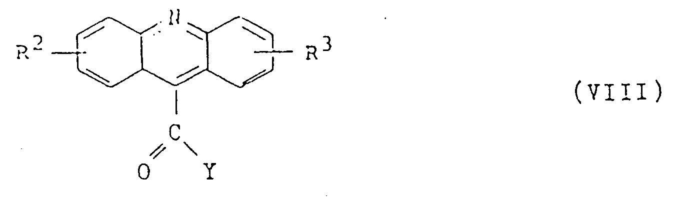 dans laquelle Y représente un atome d'halogène ou un groupe oxycarbonylalkyle(C₁-C₅), oxycarbonylaryle ou imidazolide, avec un sulfonamide primaire ou secondaire ou avec un acide thiocarboxylique de formule IX
HS-X-COOH (IX)
X ayant les significations données dans la revendication 1, et ensuite on convertit l'acide obtenu en le dérivé d'acridine recherché, contenant le radical R⁵, qui est ensuite encore rendu quaternaire au niveau de l'atome d'azote du squelette de l'acridine.

7. Procédé pour la préparation de composés comportant des groupes sulfonamido substitués de formule V selon la revendication 1, caractérisé en ce que l'on fait réagir un composé de formule VIII avec un acide sulfonamidocarboxylique protégé de formule X 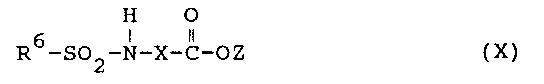 ou de formule XI 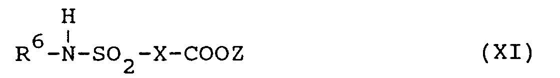 X et R⁶ ayant les significations données dans la revendication 1 et Z étant un radical protégeant le groupe carboxy, qui est ensuite éliminé, et l'acide ainsi formé est converti en le dérivé d'acridinium recherché, contenant le radical R⁵, qui est ensuite encore rendu quaternaire au niveau de l'atome d'azote du squelette de l'acridine.

8. Composé luminescent, caractérisé en ce qu'un dérivé d'acridinium selon la revendication 1 est lié directement, ou par une molécule jouant le rôle de pont, à une protéine, un polypeptide ou à une autre substance d'intérêt biologique, avec formation d'un conjugué stable, immunologiquement actif.

9. Essai immuno-enzymatique par luminescence, pour la détermination d'une substance antigénique dans un échantillon de liquide, dans lequel, dans un procédé compétitif ou en sandwich, au moins un composant immunologiquement actif est immobilisé sur une phase solide et au moins un autre composant, le marqueur, est un composé luminescent selon la revendication 8.

10. Essai immuno-enzymatique par luminescence selon la revendication 9, caractérisé en ce que
a) on met à incuber un anticorps immobilisé, réagissant spécifiquement avec un antigène, avec un échantillon du liquide à étudier et un conjugué de l'antigène et d'un dérivé d'acridinium chimiluminescent selon la revendication 1;
b) on sépare l'échantillon et le conjugué marqué non lié;
c) on met le conjugué marqué, lié, en contact avec un oxydant, afin de provoquer une émission de lumière; et
d) on détermine, à partir dc l'intensité mesurée de la lumière émise, la quantité de l'antigène présent.

11. Essai immuno-enzymatique par luminescence selon la revendication 10, caractérisé en ce que, avant l'addition du conjugué marqué, on sépare le liquide à étudier de l'anticorps immobilisé.

12. Essai immuno-enzymatique par luminescence selon la revendication 9, caractérisé en ce que
a) on met à incuber un anticorps immobilisé, réagissant spécifiquement avec l'antigène, avec un échantillon du liquide à étudier et un conjugué d'un second anticorps réagissant spécifiquement et d'un dérivé d'acridinium chimiluminescent selon la revendication 1;
b) on sépare l'échantillon et le conjugué marqué non lié;
c) on met le conjugué marqué, lié, en contact avec un oxydant, afin de provoquer une émission de lumière; et
d) on détermine, à partir de l'intensité mesurée de la lumière émise, la quantité de l'antigène présent.

13. Essai immuno-enzymatique par luminescence selon la revendication 12, caractérisé en ce que, avant l'addition du conjugué marqué, on sépare le liquide à étudier de l'anticorps immobilisé.

14. Essai immuno-enzymatique par luminescence selon la revendication 9, caractérisé en ce que
a) on met à incuber un antigène immobilisé, réagissant spécifiquement avec l'anticorps, avec un échantillon du liquide à étudier et une solution d'un conjugué de l'anticorps et d'un dérivé d'acridinium chimiluminescent selon la revendication 1;
b) on sépare l'échantillon et le conjugué marqué non lié;
c) on met le conjugué marqué, lié, en contact avec un oxydant, afin de provoquer une émission de lumière; et
d) on détermine, à partir de l'intensité mesurée de la lumière émise, la quantité de l'antigène présent.

15. Essai immuno-enzymatique par luminescence selon la revendication 9, caractérisé en ce que
a) on met à incuber un antigène immobilisé, réagissant spécifiquement avec l'anticorps, avec une solution d'un conjugué de l'anticorps et d'un dérivé d'acridinium chimiluminescent selon la revendication 1;
b) on sépare le conjugué marqué n'ayant pas réagi;
c) on ajoute un échantillon du liquide à étudier;
d) on sépare ensuite à nouveau l'échantillon;
e) on met le conjugué marqué, lié, en contact avec un oxydant, afin de provoquer une émission de lumière; et
f) on détermine, à partir de l'intensité mesurée de la lumière émise, la quantité de l'antigène présent.

16. Essai immuno-enzymatique par luminescence selon la revendication 9, caractérisé en ce que
a) on met à incuber un antigêne immobilisé, réagissant spécifiquement avec l'anticorps, avec une solution d'un conjugué de l'anticorps et d'un dérivé d'acridinium chimiluminescent selon la revendication 1;
b) on ajoute un échantillon du liquide à étudier;
c) on sépare l'échantillon et le conjugué non lié;
d) on met le conjugué marqué, lié, en contact avec un oxydant, afin de provoquer une émission de lumière; et
e) on détermine, à partir de l'intensité mesurée de la lumière émise, la quantité de l'antigène présent.
